Europäisches Patentamt

(19) European Patent Office

Office européen des brevets

(11) **EP 0 747 417 A1**

(12) **EUROPEAN PATENT APPLICATION**
published in accordance with Art. 158(3) EPC

(43) Date of publication:
  **11.12.1996 Bulletin 1996/50**

(51) Int. Cl.$^6$: **C08G 73/00**, C08G 73/10,
  C08G 69/00, C08G 69/10

(21) Application number: **95941836.9**

(22) Date of filing: **21.12.1995**

(86) International application number:
  **PCT/JP95/02623**

(87) International publication number:
  **WO 96/19524 (27.06.1996 Gazette 1996/29)**

(84) Designated Contracting States:
  **BE DE FR GB IT**

(30) Priority: **21.12.1994 JP 318638/94**
  **10.03.1995 JP 51558/95**
  **24.04.1995 JP 98492/95**
  **24.04.1995 JP 98493/95**

(71) Applicant: **MITSUBISHI CHEMICAL CORPORATION**
  **Chiyoda-ku, Tokyo 100 (JP)**

(72) Inventors:
  • **NAKATO, Takeshi**
    **Ibaraki 300-03 (JP)**
  • **KURAMOCHI, Mayumi**
    **Ibaraki 300-03 (JP)**
  • **TOMIDA, Masayuki**
    **Ibaraki 300-03 (JP)**

(74) Representative: **Kindler, Matthias, Dr. Dipl.-Chem. et al**
  **Hoffmann, Eitle & Partner,**
  **Patent- und Rechtsanwälte,**
  **Arabellastrasse 4**
  **81925 München (DE)**

(54) **POLYASPARTIC ACID OR SALT THEREOF AND PROCESS FOR PRODUCING THE SAME**

(57)    The present invention relates to polyaspartic acid or salts thereof having a calcium sequestration capacity of not less than 4.3 (Ca$^{++}$g/100 g polymer), a process for producing said polyaspartic acid or salts thereof which comprises subjecting a monomer selected from the group consisting of a reaction product obtained by reacting maleic acid with ammonia, aspartic acid and maleamic acid to a polycondensation reaction in the presence of a solvent and an acid catalyst to obtain polysuccinimide and hydrolyzing said polysuccinimide, and polysuccinimide which is a precursor of said polyaspartic acid or salts thereof.

    The present invention also relates to a process for producing polyaspartic acid or salts thereof which comprises subjecting a monomer selected from the group consisting of a reaction product obtained by reacting maleic acid with ammonia, aspartic acid and maleamic acid to a polycondensation reaction in the presence of an acid catalyst and a mixed solvent which comprises two or more solvents including at least one aprotic polar solvent to obtain polysuccinimide, and hydrolyzing said polysuccinimide.

    These polyaspartic acid or salts thereof are useful as a chelating agent, a coagulant, a scale inhibitor, a builder for detergent, a dispersing agent, a humectant, an additive for fertilizer, a row material for biodegradable polymer and the like.

**Description**

TECHNICAL FIELD

The present invention relates to polyaspartic acid or salts thereof and a process for production thereof. The polyaspartic acid or salts thereof according to the present invention is useful as a chelating agent, a scale inhibitor, a builder for detergent, a dispersing agent, a humectant, an additive for fertilizer, various coating agents and the like.

BACKGROUND ART

Conventionally, polyaspartic acid has been used as a chelating agent, a scale inhibitor, a builder for detergent, a dispersing agent, a humectant, an additive for fertilizer and the like. It can be usually obtained by hydrolyzing polysuccinimide. Commonly known methods for producing polysuccinimide include a method of reacting aspartic acid or maleamic acid on a solid phase at a high temperature of not less than 180°C (cf. U.S. Patents No. 5,057,597, No. 5,219, 986 and No. 5,315,010, and JP-A-6-206937), a method of reacting ammonia with maleic anhydride on a solid phase at a temperature of not less than 120°C (cf. U.S. Patents No. 4,839,461 and No. 5,296,578), a method of reacting aspartic acid or maleamic acid in the presence of a solvent such as polyethylene glycol, N-methylpyrrolidone or sulfolane at a temperature of not less than 120°C (cf. JP-A-6-145350 and JP-A-6-211982) and the like. According to these methods, however, it is necessary to react the starting material at a high temperature for a long period of time in order to increase a conversion rate or yield. Further, polyaspartic acid obtained by these methods has a poor calcium sequestration capacity.

As a shortened period of the reaction, a method of reacting aspartic acid in the presence of phosphoric acid or polyphosphoric acid catalyst on a solid phase at the temperature of from 100 to 250°C is known (cf. JP-B-48-20638 and U.S. Patent No. 5,142,062). However, since this method requires the use of a large amount of the catalyst for obtaining polysuccinimide, there are problems that complicated procedure is necessary to remove the large amount of the catalyst in the post-treatment, that an apparatus to be used for the reaction must be resistant to corrosion and that it is difficult to control the molecular weight of polysuccinimide to be obtained. Furthermore, since the reaction is carried out on a solid phase in these production methods, there arises a problem that polymer aggregates to form a lump thereof by polymerization during the production process, whereby being difficult to apply these methods to industrial production.

DESCRIPTION OF THE INVENTION

An object of the present invention is to provide polyaspartic acid or salts thereof which is useful as a chelating agent, a scale inhibitor, a builder for detergent, a dispersing agent, a humectant, an additive for fertilizer, various coating agents and the like, and to provide a process for producing said compound simply with high yield.

In view of the above problems, the present inventors conducted intensive study and, as a result, it has been found that polyaspartic acid having a high calcium sequestration capacity can be obtained by subjecting a monomer selected from the group consisting of a reaction product obtained by reacting maleic acid with ammonia, aspartic acid and maleamic acid to polycondensation reaction in the presence of a solvent and an acid catalyst. It has also been found that high molecular weight polysuccinimide, which cannot be obtained by the conventional solvent method, can be obtained and by using this compound various polyaspartic acid species having widely varied molecular weight can be simply produced. Thus, the present invention has been completed.

Accordingly, the present invention provides polyaspartic acid or salts thereof having a calcium sequestration capacity of not less than 4.3 ($Ca^{++}g/100$ g polymer) (hereinafter referred to as "the first polyaspartic acid or salts thereof"), a process for producing said polyaspartic acid or salts thereof which comprises a polycondensation step in which a monomer selected from the group consisting of a reaction product obtained by reacting maleic acid with ammonia, aspartic acid and maleamic acid is subjected to a polycondensation reaction in the presence of a solvent and an acid catalyst to obtain polysuccinimide and a hydrolysis step in which the resulting polysuccinimide is hydrolyzed (hereinafter referred to as "the first process for producing polyaspartic acid or salts thereof"), and polysuccinimide which is a precursor of said polyaspartic acid or salts thereof and can be hydrolyzed to give said aspartic acid or salts thereof.

The present invention also provides a process for producing aspartic acid or salts thereof which comprises a polycondensation step in which a monomer selected from the group consisting of a reaction product obtained by reacting maleic acid with ammonia, aspartic acid and maleamic acid is subjected to a polycondensation reaction in the presence of an acid catalyst and a mixed solvent which comprises two or more solvents including at least one aprotic polar solvent to obtain polysuccinimide and a hydrolysis step in which the polysuccinimide obtained in the above polycondensation step is hydrolyzed (hereinafter referred to as "the second process for producing polyaspartic acid or salts thereof).

The present invention will be described in detail below.

(1) First polyaspartic acid or salts thereof and process for production thereof

The first polyaspartic acid or salts thereof according to the present invention has a calcium sequestration capacity determined by the calcium ion electrode method using calcium chloride of not less than 4.3 ($Ca^{++}g/100$ g polymer), preferably not less than 4.6 ($Ca^{++}g/100$ g polymer). The calcium sequestration capacity of the polyaspartic acid or salts thereof shows low dependence on molecular weight. It is generally known that the calcium sequestration capacity increases as the molecular weight becomes higher, but those having too high molecular weight are not preferred since their dispersibility lowers. The calcium sequestration capacity of the first polyaspartic acid or salts thereof according to the present invention shows a low molecular weight-dependence, and therefore, it has a high calcium sequestration capacity as described above even if its molecular weight is comparatively low.

The term "calcium sequestration capacity" used herein means a value calculated from the result obtained by adding and dissolving a weighed sample into an aqueous solution containing calcium chloride and potassium chloride followed by stirring and measuring an amount of calcium ions in the solution using an ion meter having a calcium ion electrode in accordance with the method as described in, for example, JP-A-5-59130 or J. Jpn. Oil Chem. Soc. (YUKAGAKU), Vol.35, No.3 (1986). More specifically, it means an amount of calcium ions in terms of g captured by 100 g of a sample polymer (unit: $Ca^{++}g/100$ g polymer), which is determined by weighing and dissolving 10 mg of a sample in 50 ml of an aqueous solution, which has been adjusted to have a calcium chloride concentration of $1.0 \times 10^{-3}$ M and a potassium chloride concentration of 0.08 M, contained in a 50-ml beaker and stirring the resulting mixture at 30°C for about 10 minutes to measure calcium ions in the solution using a calcium ion electrode (Orion Model 93-20) and an ion meter (Orion Model 720A). Alternatively, a calcium sequestration capacity may be determined by the other measuring method such as a chromogen method or a turbidity method.

Examples of the polyaspartic acid salts include alkali metal salts such as sodium polyaspartate salt or potassium polyaspartate salt, alkaline earth metal salts such as calcium polyaspartate salt or magnesium polyaspartate salt, and the like.

The first polyaspartic acid or salts thereof according to the present invention having such a high calcium sequestration capacity can be produced by a process comprising a polycondensation step in which a monomer selected from the group consisting of a reaction product obtained by reacting maleic acid with ammonia, aspartic acid and maleamic acid is subjected to a polycondensation reaction in the presence of a solvent and an acid catalyst to obtain polysuccinimide and a hydrolysis step in which polysuccinimide obtained in the polycondensation step is hydrolyzed.

The monomer used in the first process for producing polyaspartic acid or salts thereof is selected from the group consisting of a reaction product obtained by reacting maleic acid with ammonia, aspartic acid and maleamic acid.

The reaction product obtained by reacting maleic acid with ammonia means a product obtained by reacting maleic acid with ammonia in accordance with the method described in, for example, German Patent No. 3,626,672, U.S. Patents No. 4,839,461 and No. 5,286,810 and the like. More specifically, it is exemplified by the method described in Production Example 1 in the item of "Best Mode for Practice of the Invention" given below. Incidentally, maleic acid to be used as a starting material of the above reaction product may be in the form of anhydride, partial ester or complete ester. Further, ammonia can be generally used in the form of gas or a solution. In case of using its solution, ammonia may be dissolved in water to give an ammonium hydroxide aqueous solution or dissolved in alcohol such as methanol or ethanol or in other appropriate organic solvent.

The above-described reaction product is mainly a maleic acid monoammonium salt, and it may include other products such as maleic acid, a maleic acid diammonium salt, ammonia, fumaric acid, aspartic acid, asparagine, iminodisuccinic acid, maleamic acid and the like.

Aspartic acid may be D-form, L-form or a mixture thereof. Maleamic acid can be obtained by, for example, heating a monoammonium salt or a diammonium salt of maleic acid.

Among these monomers, aspartic acid is preferred.

As the monomer in addition to the above-described reaction product obtained by reacting maleic acid with ammonia, aspartic acid and maleamic acid, the other monomer capable of copolymerization may be used provided that its amount does not exceed 50 mol% of the total monomer amount. Examples thereof include amino acid or salts thereof such as an aspartic acid salt, glutamic acid, alanine, leucine or lysine, hydroxycarboxylic acid such as glycolic acid, lactic acid or 3-hydroxybutyric acid, and a compound having at least one functional group reactive with an amino group and/or a carboxyl group such as 2-hydroxyethanol, maleic acid or aniline.

Any solvent can be used as a solvent used in the polycondensation step in the production process of the present invention as far as it is an organic solvent usually used for polycondensation. It is preferable to use a solvent having a boiling point of not less than 100°C, particularly not less than 130°C. Such a solvent is preferably selected from the group consisting of hydrocarbon type solvents, halogenated hydrocarbon type solvents, ether type solvents, ester type solvents and aprotic polar solvents.

Specific examples of the hydrocarbon type solvents include xylene, diethylbenzene (these may be an ortho-, meta- or para-isomer alone or a mixture of two or more of the isomers), toluene, amylbenzene, cumene, mesitylene, tetralin and the like. Examples of the halogenated hydrocarbon type solvents include chlorotoluene, dichlorobenzene (these may be an ortho-, meta- or para-isomer alone or a mixture of two or more of the isomers), 1,4-dichlorobutane, chlorobenzene and the like. Examples of the ether type solvents include dichloroethylether, butyl ether, diisoamyl ether, anisole and the like. Examples of the ester type solvents include n-amyl acetate, isoamyl acetate, methylisoamyl acetate, methoxybutyl acetate, cyclohexyl acetate, benzyl acetate, n-butyl propionate, isoamyl propionate, isoamyl butylate, n-butyl butylate and the like. Examples of the aprotic polar solvents include an amide type solvent such as N,N-dimethylformamide, N,N-dimethyl-acetamide, N-methyl-2-pyrrolidone, 1,3-dimethyl-2-imidazolidinone or tetramethyluric acid, a sulfur-containing solvent such as dimethylsulfoxide or sulfolane, a phosphorus-containing solvent such as hexamethylphosphoroamide, and the like. Among these, preferred are those having a moderate boiling point such as diethylbenzene, mesitylene, cumene, chlorotoluene, 1,4-dichlorobutane, diisoamyl ether, isoamyl butylate, 1,3-dimethyl-2-imidazolidinone or sulfolane. Particularly preferred are mesitylene, cumene, chlorotoluene, 1,3-dimethyl-2-imidazolidinone and sulfolane. These solvents may be used alone or as a mixture of two or more thereof.

In the production process of the present invention, the solvent can be used in an amount of usually from 100 to 5,000 parts by weight, preferably from 200 to 2,000 parts by weight, based on 100 parts by weight of the monomer.

Examples of the acid catalyst used in the polycondensation step include an inorganic acid catalyst such as sulfuric acid, sulfuric anhydride, phosphoric acid, polyphosphoric acid, metaphosphoric acid or fused phosphoric acid, and an organic acid catalyst such as p-toluenesulfonic acid, trichloroacetic acid, trifluoroacetic acid or trifluoromethanesulfonic acid. Among these, it is preferable to use phosphoric acid which is a weak acid. The amount of the acid catalyst to be used ranges generally from 0.0002 to 2.0 mole, preferably from 0.01 to 1.0 mole, more preferably from 0.01 to 0.5 mole, most preferably 0.03 to 0.3 mole, per mole of the monomer. Thus, according to this production process, the polycondensation reaction can be effected sufficiently using a smaller amount of the acid catalyst, as compared with the conventional methods. Therefore, it is possible to prevent aggregation of the polymer to form a lump thereof by polymerization and the process is advantageously applied to the industrial production.

The reaction temperature in the polycondensation reaction of the production process of the present invention ranges generally from 100 to 280°C, preferably from 130 to 250°C, more preferably from 150 to 200°C. When the polycondensation temperature is lower than 100°C, the reaction does not easily proceed. When the temperature exceeds 280°C, a decomposition product may possibly formed. There is no limitation to the pressure during the reaction and the reaction can be effected under ordinary pressure, reduced pressure or pressurized conditions. Generally, the pressure ranges from 10 Pa to 1 MPa. The reaction period ranges from 1 minute to 100 hours, preferably from 10 minutes to 50 hours, most preferably from 15 minutes to 20 hours. The reaction is regarded as substantially completed when azeotrope of water produced during the reaction as a side product ends.

After the completion of the polycondensation reaction, a post-treatment is carried out to recover polysuccinimide obtained by the polycondensation reaction. An appropriate post-treatment can be selected depending on usage of the obtained polysuccinimide. For example, polysuccinimide can be recovered by the method conventionally used such as the method of removing the solvent by filtration or centrifugation of the polycondensation reaction product and, the method further comprising, after the solvent is removed, washing the residue with water or a solvent having a low boiling point such as methanol or acetone.

Polysuccinimide thus obtained is a precursor of the first polyaspartic acid or salts thereof according to the present invention, and is hydrolyzed to give the desired polyaspartic acid or salts thereof. Further, this polysuccinimide per se can be used as a lubricant for polyurethane, various coating agents and the like.

The first polyaspartic acid or salts thereof according to the present invention can be obtained by subsequently hydrolyzing polysuccinimide obtained by the above polycondensation step. The hydrolysis step of polysuccinimide can be carried out by the method commonly used. Typical examples thereof are the methods as described in J. Am. Chem. Soc. 80, 3361 (1958), J. Org. Chem 26, 1084 (1961), U.S. Patents No. 5,221,733 and No. 5,288,783, JP-A-60-203636 and the like. More specifically, these methods are hydrolysis methods using metal hydroxide such as sodium hydroxide and the like. Using this method, polyaspartic acid or salts thereof having an excellent calcium sequestration capacity can be produced.

The molecular weight of the first polyaspartic acid or salts thereof according to the present invention thus obtained is not particularly limited, but it ranges preferably from 500 to 200,000, particularly from 4,000 to 100,000, in terms of weight average molecular weight calculated based on polyethylene glycol.

As described above, the calcium sequestration capacity of the first polyaspartic acid or salts thereof according to the present invention shows low dependence on molecular weight and thus, even those having comparatively low molecular weight have a high calcium sequestration capacity. In view of dispersibility, it is preferable to use the compound having weight average molecular weight calculated based on polyethylene glycol of from 500 to 30,000, more preferably from 1,000 to 20,000.

The first polyaspartic acid or salts thereof according to the present invention can be suitably applied to various usages including a chelating agent, a scale inhibitor, a builder for detergent, a dispersing agent, a humectant, an additive for fertilizer, various coating agents and the like.

(2) Second process for producing polyaspartic acid or salts thereof

The present invention also provides a second process for producing the polyaspartic acid or salts thereof which comprises a polycondensation step in which a monomer selected from the group consisting of a reaction product obtained by reacting maleic acid with ammonia, aspartic acid and maleamic acid is subjected to a polycondensation reaction in the presence of an acid catalyst and a mixed solvent containing two or more solvents and at least one aprotic polar solvent to obtain polysuccinimide, and a hydrolysis step in which polysuccinimide obtained in the above polycondensation step is hydrolyzed.

It is not easy to produce polysuccinimide having a high molecular weight simply and conveniently by the conventional method. On the other hand, the process of the present invention enables the production of high molecular weight polysuccinimide having weight average molecular weight of, for example, not less than about 25,000 calculated based on polystyrene, which is hydrolyzed to give high molecular weight polyaspartic acid or salts thereof having an excellent calcium sequestration capacity.

The monomer used in this process is the same as those used in the first process for producing polyaspartic acid or salts thereof, whereas a mixed solvent which comprises two or more solvents including at least one aprotic polar solvent is used as a solvent in the second process for producing polyaspartic acid or salts thereof. The mixed solvent includes preferably an aprotic polar solvent and a solvent selected from the group consisting of hydrocarbon type solvents, halogenated hydrocarbon type solvents, ether type solvents and ester type solvents. Each of the solvents used in the mixed solvent has a boiling point of preferably not less than 100°C, more preferably not less than 130°C. The content rate of the aprotic polar solvent ranges generally from 1 to 99 wt%, preferably from 5 to 99 wt%, more preferably from 10 to 95%, based on the total weight of the mixed solvent. It should be noted that examples of the respective solvents are the same as those described in the first process for producing polyaspartic acid or salts thereof.

By using such a mixed solvent, polysuccinimide having higher molecular weight can be obtained in the polycondensation step of the second process for producing polyaspartic acid or salts thereof according to the present invention. This process is industrially useful since it is possible to prevent aggregation of the polymer to form a lump thereof by polymerization, without using a large quantity of an acid catalyst. The acid catalyst is used in an amount of generally from 0.0002 to 2.0 mole, preferably from 0.01 to 1.0 mole, more preferably from 0.01 to 0.5 mole, and most preferably from 0.03 to 0.3 mole, per mole of the monomer, like in the first process of producing polyaspartic acid or salts thereof.

The second process for producing polyaspartic acid or salts thereof according to the present invention is carried out under the same conditions including the polycondensation reaction temperature, pressure, reaction period, post-treatment, and the like as in the first process for producing polyaspartic acid or salts thereof.

In the polycondensation step of the second process for producing polyaspartic acid or salts thereof, polycondensation can be carried out in the presence of primary amine or secondary amine as well as the above-described solvents and the acid catalyst. The addition of amine makes it possible to control the molecular weight of polysuccinimide and to obtain polysuccinimide having desired molecular weight depending on its usage, thereby capable of producing polyaspartic acid or salts thereof having desired molecular weight.

The primary amine or secondary amine used herein is not particularly limited, but it preferably has an acid dissociation constant (pKa) in an aqueous solution of preferably not more than 7, more preferably not more than 6. The term "pKa" used herein means a value of conjugate acid of amine, for example, described as the dissociation constant of organic compounds in an aqueous solution in "Kagaku Binran, Kisohen (Chemical Survey, Basics)" (ed. by Japanese Society of Chemistry) or described in "Critical Stability Constants, Vols. 1, 2, 3 and 5" (A.E. Martell, R.M. Smith, Plenum Press). Specific examples thereof include morpholine, hexylamine, o-, m- and p-aminopyridine, butylamine, aniline, triethylene tetramine, 1-naphthylamine, 2-naphthylamine, and the like. O-Aminophenol and 4-aminobutyric acid, which are bifunctional, may also be exemplified. Among these, the amines having pKa of not more than 6 are aniline, triethylene tetramine, 1-naphthylamine, 2-naphthylamine, and the like, and as a bifunctional compound, o-aminophenol and 4-aminobutyric acid. These amines may be used alone or in combination of two or more thereof.

The method of addition of the primary amine or secondary amine in the polycondensation step is not particularly limited. It may be added in a whole amount at the time of commencement of the polycondensation reaction or may be added gradually with the course of time during the polycondensation reaction. The amount of the amine may be varied depending on its kind and desired molecular weight of polysuccinimide. Thus, a larger amount of the amine may be used when polysuccinimide having lower molecular weight is desired. Specifically, it is used in an amount of generally not more than 100 mol%, preferably not more than 80 mol%, more preferably not more than 60 mol%, based on the monomer. Incidentally, the amine is considered to function as a polymer end-blocking agent. In other words, it is considered that it reacts with a carboxylic acid at the end of the polymer to prevent the reaction of the monomer with the active end of the polymer, thereby controlling its molecular weight.

The polysuccinimide obtained in the polycondensation step is a precursor of the second polyaspartic acid or salts thereof, which is hydrolyzed to give the polyaspartic acid or salts thereof. This polysuccinimide per se can be used as a lubricant for polyurethane, a row material for biodegradable polymer and the like.

The second polyaspartic acid or salts thereof according to the present invention can be obtained by subsequently hydrolyzing polysuccinimide obtained in the polycondensation step in the same manner as in the case of the first polyaspartic acid or salts thereof.

The second polyaspartic acid or salts thereof according to the present invention can be applied to various usages such as a coagulant, a row material for biodegradable polymer or the like.

## BEST MODE FOR THE PRACTICE OF THE INVENTION

The present invention will be described in more detail below with reference to Examples, but is not construed to be limited to these Examples at all.

It should be noted that the conversion rate (%) of aspartic acid into polysuccinimide was determined by dissolving 10 g of a reaction mixture or a reaction product in 200 g of N,N-dimethylformamide (DMF) under stirring for 4 hours, filtering off aspartic acid which does not dissolve in the solvent (DMF-insoluble residue) and weighing it to apply to the following equation.

$$\text{Conversion rate (\%)} = \frac{10 - (\text{DMF-insoluble residue})}{10} \times 100$$

The molecular weight of polysuccinimide thus produced was determined by GPC chromatography (differential refractometer) using a TSK gel "GMHHR-M" column and a TSK gel "G2000HHR" column (both manufactured by Tosoh Corporation) and a solution of 10 mM lithium bromide in dimethylformamide as an eluent and calculated based on polystyrene. The molecular weight of polyaspartic acid or salts thereof was determined by GPC chromatography (differential refractometer) using a TSK gel "G6000PWXL" column (Tosoh Corporation) and a TSK gel "G3000PWXL" column (Tosoh Corporation) in the case that polysuccinimide has weight average molecular weight of not less than 30,000 and two TSK gel "G3000PWXL" columns (Tosoh Corporation) in the case that polysuccinimide has weight average molecular weight of lower than 30,000, and a 0.4 M sodium nitrate aqueous solution as an eluent and calculated based on polyethylene glycol.

As a calcium sequestration capacity, a calcium chloride-derived calcium ion-capturing ability per 100 g of the polymer was determined using a calcium ion electrode and an ion meter in accordance with the description of, for example, JP-A-5-59130 and J. Jpn. Oil Chem. Soc. (YUKAGAKU), 35 (3) (1986). More specifically, 10 mg of a sample was weighed and dissolved in 50 ml of an aqueous solution, which had been adjusted to give a calcium chloride concentration of $1.0 \times 10^{-3}$ M and a potassium chloride concentration of 0.08 M, contained in a 50-ml beaker, the resulting mixture was stirred at 30°C for about 10 minutes to determine calcium ions in the solution using a calcium ion electrode (Orion Model 93-20) and an ion meter (Orion Model 720A) in terms of g of the calcium ions captured by 100 g of the sample.

## PRODUCTION EXAMPLE 1

(Production of Reaction Product of Maleic Acid and Ammonia)

98 g of maleic anhydride and 50 g of water were placed into a 300-ml four-neck flask equipped with a reflux condenser, a thermometer, a stirrer and a dropping funnel, and were heated for 30 minutes in an oil bath at 75°C. Succeedingly, the flask was taken out from the oil bath and 68.1 g of a 25% aqueous ammonia was added dropwise to the flask under ice-cooling. The resulting mixture was further stirred in an oil bath at 85°C for 3 hours. After the reaction, water was distilled off from the reaction mixture at 80 to 85°C under reduced pressure (200 to 10 hPa) to obtain 130 g of a white solid.

## EXAMPLE 1

Into a 200-ml four-neck flask equipped with a reflux condenser, a thermometer, a stirrer and a Dean-Stark trap, 25 g of the reaction product of maleic acid and ammonia obtained in Production Example 1 as a starting material, 2.5 g of 85% phosphoric acid as an acid catalyst and 80 g of mesitylene as a solvent were placed. The resulting mixture was refluxed (162 °C) for 4.5 hours under ordinary pressure to carry out a polycondensation reaction. Water formed during the reaction was distilled off outside the system together with the solvent.

After the completion of the reaction, the reaction product was collected by filtration and washed with 100 g of pure water four times and subsequently with 100 g of methanol. The resulting reaction product was dried at 80°C for 24 hours under reduced pressure to obtain 13.6 g of polysuccinimide in the form of brown powder.

The conversion rate of polysuccinimide thus obtained was 99%. Its molecular weight determined by GPC and calculated based on polystyrene was 4,500 in terms of weight average molecular weight and 3,500 in terms of number average molecular weight.

Into a 100-ml beaker with a stirring bar were placed 3 g of polysuccinimide obtained above and 10 g of water. An aqueous solution of 1.4 g of sodium hydroxide in 20 g of water was added thereto under ice-cooling followed by stirring for 1 hour to carry out hydrolysis of polysuccinimide. After the reaction, the reaction mixture was crystallized by pouring into 300 ml of methanol to obtain 3.3 g of yellowish white sodium polyaspartate.

Its molecular weight determined by GPC and calculated based on polyethylene glycol was 4,200 in terms of weight average molecular weight and 3,600 in terms of number average molecular weight. Its calcium sequestration capacity was 5.0 ($Ca^{++}g/100$ g polymer).

EXAMPLE 2

Into a 200-ml four-neck flask equipped with a reflux condenser, a thermometer, a stirrer, a nitrogen-introducing tube and a Dean-Stark trap were placed 25 g of the reaction product of maleic acid and ammonia obtained in Production Example 1 as a starting material, 2.5 g of 85% phosphoric acid as an acid catalyst and 80 g of sulfolane as a solvent. Subsequently, the resulting mixture was subjected to a polycondensation reaction with maintaining the reaction temperature at 180 °C for 4.5 hours under ordinary pressure. Water formed during the reaction was distilled off outside the system together with a stream of nitrogen.

After the completion of the reaction, the reaction product was crystallized from 300 g of pure water and collected by filtration. The resulting crystals were washed with 100 g of pure water four times and subsequently with 100 g of methanol. The resulting reaction product was dried at 80°C for 24 hours under reduced pressure to obtain 10.1 g of polysuccinimide in the form of yellowish white powder. The conversion rate of polysuccinimide thus obtained was 99% or higher. Its molecular weight determined by GPC and calculated based on polystyrene was 6,000 in terms of weight average molecular weight and 4,800 in terms of number average molecular weight.

Polysuccinimide obtained above was hydrolyzed in the same manner as in Example 1 to obtain 3.1 g of yellowish white sodium polyaspartate. Its molecular weight determined by GPC and calculated based on polyethylene glycol was 5,800 in terms of weight average molecular weight and 4,600 in terms of number average molecular weight. Its calcium sequestration capacity was 5.1 ($Ca^{++}g/100$ g polymer).

EXAMPLE 3

Using 25 g of maleamic acid as a staring material, 2.5 g of 85% phosphoric acid as an acid catalyst, 56 g of mesitylene and 24 g of sulfolane as solvents, the reaction was carried out in the same manner as in Example 1 to obtain 12.9 g of polysuccinimide in the form of powder.

The conversion rate of polysuccinimide thus obtained was 99%. Its molecular weight determined by GPC and calculated based on polystyrene was 7,100 in terms of weight average molecular weight and 4,800 in terms of number average molecular weight.

Subsequently, this polysuccinimide was hydrolyzed in the same manner as in Example 1 to obtain 3.4 g of yellowish white sodium polyaspartate.

Its molecular weight determined by GPC and calculated based on polyethylene glycol was 6,900 in terms of weight average molecular weight and 4,600 in terms of number average molecular weight. Its calcium sequestration capacity was 5.4 ($Ca^{++}g/100$ g polymer).

EXAMPLE 4

The reaction was carried out in the same manner as in Example 3 except for using 56 g of n-butyl butylate as a solvent in place of 56 g of mesitylene to obtain 13.7 g of polysuccinimide powder. The conversion rate of polysuccinimide thus obtained was 99% or higher. Its molecular weight determined by GPC and calculated based on polystyrene was 7,200 in terms of weight average molecular weight and 4,800 in terms of number average molecular weight.

This polysuccinimide was hydrolyzed in the same manner as in Example 5 to obtain 3.2 g of yellowish white sodium polyaspartate. Its molecular weight determined by GPC and calculated based on polyethylene glycol was 6,800 in terms of weight average molecular weight and 4,600 in terms of number average molecular weight. Its calcium sequestration capacity was 4.8 ($Ca^{++}g/100$ g polymer).

EXAMPLE 5

Using 25 g of aspartic acid as a staring material, 2.5 g of 85% phosphoric acid as an acid catalyst, 80 g of p-chlorotoluene as a solvent, the reaction was carried out at 164°C in the same manner as in Example 1 to obtain 17.4 g of polysuccinimide in the form of yellowish white powder. The conversion rate of polysuccinimide thus obtained was 99% or higher. Its molecular weight determined by GPC and calculated based on polystyrene was 19,000 in terms of weight average molecular weight and 11,000 in terms of number average molecular weight.

This polysuccinimide was hydrolyzed in the same manner as in Example 1 to obtain 3.8 g of yellowish white sodium polyaspartate. Its molecular weight determined by GPC and calculated based on polyethylene glycol was 19,000 in terms of weight average molecular weight and 12,000 in terms of number average molecular weight. Its calcium sequestration capacity was 4.63 ($Ca^{++}$g/100 g polymer).

EXAMPLE 6

Using 25 g of aspartic acid as a staring material, 2.5 of 85% phosphoric acid as an acid catalyst, 80 g of sulfolane as a solvent, the reaction was carried out at 180°C in the same manner as in Example 1. After the completion of the reaction, the reaction product was crystallized from 300 g of pure water, collected by filtration and washed with 100 g of pure water four times and subsequently with 100 g of methanol. The resulting reaction product was dried at 80°C for 24 hours under reduced pressure to obtain 17.1 g of yellowish white powder of polysuccinimide. The conversion rate of polysuccinimide thus obtained was 99% or higher. Its molecular weight determined by GPC and calculated based on polystyrene was 14,000 in terms of weight average molecular weight and 9,000 in terms of number average molecular weight.

This polysuccinimide was hydrolyzed in the same manner as in Example 1 to obtain 3.6 g of yellowish white sodium polyaspartate. Its molecular weight determined by GPC and calculated based on polyethylene glycol was 15,000 in terms of weight average molecular weight and 9,800 in terms of number average molecular weight. Its calcium sequestration capacity was 5.0 ($Ca^{++}$g/100 g polymer).

EXAMPLE 7

Using 25 g of aspartic acid as a staring material, 2.5 g of 85% phosphoric acid as an acid catalyst, 56 g of mesitylene and 24 g of sulfolane as a solvent, the reaction was carried out in the same manner as in Example 1 to obtain 17.9 g of polysuccinimide in the form of yellowish white powder. Its yield to the theoretical value was 98%. The molecular weight of polysuccinimide determined by GPC and calculated based on polystyrene was 66,000 in terms of weight average molecular weight and 25,000 in terms of number average molecular weight.

This polysuccinimide was hydrolyzed in the same manner as in Example 1 to obtain 4.0 g of yellowish white sodium polyaspartate. Its molecular weight determined by GPC and calculated based on polyethylene glycol was 72,000 in terms of weight average molecular weight and 28,000 in terms of number average molecular weight. Its calcium sequestration capacity was 5.0 ($Ca^{++}$g/100 g polymer).

EXAMPLE 8

Using 25 g of aspartic acid as a staring material, 2.5 g of 85% phosphoric acid as an acid catalyst, 40 g of p-chlorotoluene and 40 g of sulfolane as solvents, the reaction was carried out in the same manner as in Example 1 to obtain 16.9 g of polysuccinimide in the form of yellowish white powder. Its yield to the theoretical value was 93%. The molecular weight of polysuccinimide determined by GPC and calculated based on polystyrene was 59,000 in terms of weight average molecular weight and 27,000 in terms of number average molecular weight.

This polysuccinimide was hydrolyzed in the same manner as in Example 1 to obtain 4.0 g of yellowish white sodium polyaspartate. Its molecular weight determined by GPC and calculated based on polyethylene glycol was 64,000 in terms of weight average molecular weight and 25,000 in terms of number average molecular weight. Its calcium sequestration capacity was 5.1 ($Ca^{++}$g/100 g polymer).

EXAMPLE 9

The reaction was carried out in the same manner as in Example 7 except for using 24 g of 1,3-dimethyl-2-imidazolidinone as a solvent in place of 24 g of sulfolane to obtain 17.3 g of polysuccinimide in the form of yellowish white powder. Its yield to the theoretical value was 95%. The molecular weight of polysuccinimide determined by GPC and calculated based on polystyrene was 42,000 in terms of weight average molecular weight and 23,000 in terms of number average molecular weight.

This polysuccinimide was hydrolyzed in the same manner as in Example 1 to obtain 3.7 g of yellowish white sodium polyaspartate. Its molecular weight determined by GPC and calculated based on polyethylene glycol was 46,000 in terms of weight average molecular weight and 18,000 in terms of number average molecular weight. Its calcium sequestration capacity was 5.0 ($Ca^{++}$g/100 g polymer).

EXAMPLE 10

The reaction was carried out in the same manner as in Example 8 except for using 3.6 g of p-toluenesulfonic acid as an acid catalyst in place of 2.5 g of 85% phosphoric acid to obtain 17.3 g of polysuccinimide in the form of yellowish white powder. Its yield to the theoretical value was 95%. The molecular weight of the polysuccinimide determined by GPC and calculated based on polystyrene was 41,000 in terms of weight average molecular weight and 23,000 in terms of number average molecular weight.

This polysuccinimide was hydrolyzed in the same manner as in Example 1 to obtain 3.7 g of yellowish white sodium polyaspartate. Its molecular weight determined by GPC and calculated based on polyethylene glycol was 42,000 in terms of weight average molecular weight and 17,000 in terms of number average molecular weight. Its calcium sequestration capacity was 4.9 ($Ca^{++}$g/100 g polymer).

EXAMPLE 11

The reaction was carried out in the same manner as in Example 7 except for using 56 g of diisoamyl ether as a solvent in place of 56 g of mesitylene to obtain 17.8 g of polysuccinimide in the form of yellowish white powder. Its yield to the theoretical value was 98%. The molecular weight of polysuccinimide determined by GPC and calculated based on polystyrene was 35,000 in terms of weight average molecular weight and 20,000 in terms of number average molecular weight.

This polysuccinimide was hydrolyzed in the same manner as in Example 1 to obtain 3.6 g of yellowish white sodium polyaspartate. Its molecular weight determined by GPC and calculated based on polyethylene glycol was 36,000 in terms of weight average molecular weight and 15,000 in terms of number average molecular weight. Its calcium sequestration capacity was 4.9 ($Ca^{++}$g/100 g polymer).

EXAMPLE 12

The reaction was carried out in the same manner as in Example 7 except for using 0.18 g of aniline in addition to the other materials. As a result, 17.8 g of polysuccinimide was obtained in the form of yellowish white powder. Its yield to the theoretical value was 98%. The molecular weight of polysuccinimide thus obtained was 61,000 in terms of weight average molecular weight and 23,000 in terms of number average molecular weight. This polysuccinimide was hydrolyzed in the same manner as in Example 1 to obtain sodium polyaspartate.

EXAMPLE 13

The reaction was carried out in the same manner as in Example 7 except for using 0.87 g of aniline in addition to the other materials. As a result, 18.0 g of polysuccinimide was obtained in the form of yellowish white powder. Its yield to the theoretical value was 99%. The molecular weight of polysuccinimide thus obtained was 29,000 in terms of weight average molecular weight and 10,000 in terms of number average molecular weight. Polysuccinimide thus obtained was subjected to $^1$H-NMR and, as a result, a peak corresponding to aniline was observed. Thus, it was confirmed that aniline was incorporated into the polysuccinimide molecule. This polysuccinimide was hydrolyzed in the same manner as in Example 1 to obtain sodium polyaspartate.

EXAMPLE 14

The reaction was carried out in the same manner as in Example 7 except for using 0.17 g of morpholine in addition to the other materials. As a result, 17.7 g of polysuccinimide was obtained in the form of yellowish white powder. Its yield to the theoretical value was 97%. The molecular weight of polysuccinimide thus obtained was 63,000 in terms of weight average molecular weight and 24,000 in terms of number average molecular weight. This polysuccinimide was hydrolyzed in the same manner as in Example 1 to obtain sodium polyaspartate.

EXAMPLE 15

The reaction was carried out in the same manner as in Example 7 except for using 0.82 g of morpholine in addition to the other materials. As a result, 17.6 g of polysuccinimide was obtained in the form of yellowish white powder. Its yield to the theoretical value was 97%. The molecular weight of polysuccinimide thus obtained was 32,000 in terms of weight average molecular weight and 13,000 in terms of number average molecular weight. This polysuccinimide was hydrolyzed in the same manner as in Example 1 to obtain sodium polyaspartate.

EXAMPLE 16

The reaction was carried out in the same manner as in Example 7 except for using 0.92 g of cyclohexylamine in addition to the other materials. As a result, 17.3 g of polysuccinimide was obtained in the form of yellowish white powder. Its yield to the theoretical value was 95%. The molecular weight of polysuccinimide thus obtained was 40,000 in terms of weight average molecular weight and 17,000 in terms of number average molecular weight. This polysuccinimide was hydrolyzed in the same manner as in Example 1 to obtain sodium polyaspartate.

COMPARATIVE EXAMPLE 1

98 g of maleic anhydride and 50 g of water were placed into a 300-ml four-neck flask equipped with a reflux condenser, a thermometer, a stirrer and a dropping funnel. The resulting mixture was heated for 30 minutes in an oil bath at 75°C. Thereafter, the flask was taken out from the oil bath and 68.1 g of 25% aqueous ammonia was added dropwise to the flask under ice-cooling. The resulting mixture was further stirred in an oil bath at 85°C for 3 hours. The reaction mixture was transferred to a 500-ml eggplant flask and water was removed using a rotary evaporator at 80 to 85°C under reduced pressure (200 to 10 hPa). Then, a polycondensation reaction was carried out at 120°C for 1 hour under ordinary pressure in a stream of nitrogen and further at 135°C for 4 hours under reduced pressure (20 to 10 hPa) to obtain 106 g of a white solid.

The conversion rate of polysuccinimide thus obtained was 87%. Its molecular weight determined by GPC and calculated based on polystyrene was 2,900 in terms of weight average molecular weight and 2,400 in terms of number average molecular weight.

Polysuccinimide thus obtained was hydrolyzed in the same manner as in Example 1 to obtain 1.3 g of yellowish white sodium polyaspartate. Its molecular weight determined by GPC and calculated based on polyethylene glycol was 2,500 in terms of weight average molecular weight and 2,400 in terms of number average molecular weight. Its calcium sequestration capacity was 2.4 ($Ca^{++}g/100$ g polymer).

COMPARATIVE EXAMPLE 2

Into a 200-ml eggplant flask was placed 10 g of the reaction product of maleic acid and ammonia obtained in Production Example 1. A polycondensation reaction was carried out by heating it using a rotary evaporator in an oil bath at 220°C for 4 hours in a stream of nitrogen to obtain 6.9 g of polysuccinimide in the form of brown powder. The conversion rate of polysuccinimide thus obtained was 99% or higher. Its molecular weight determined by GPC and calculated based on polystyrene was 6,500 in terms of weight average molecular weight and 4,400 in terms of number average molecular weight.

Polysuccinimide thus obtained was hydrolyzed in the same manner as in Example 1 to obtain 3.5 g of yellowish white sodium polyaspartate. Its molecular weight determined by GPC and calculated based on polyethylene glycol was 6,500 in terms of weight average molecular weight and 3,500 in terms of number average molecular weight. Its calcium sequestration capacity was 3.4 ($Ca^{++}g/100$ g polymer).

COMPARATIVE EXAMPLE 3

200 g of aspartic acid was placed into a 500-ml four-neck flask equipped with a reflux condenser, a thermometer, a stirrer and a Dean-Stark trap. A polycondensation reaction was carried out by heating the materials using a rotary evaporator in an oil bath maintained at 260°C for 6 hours in a stream of nitrogen. After the completion of the reaction, 140.0 g of polysuccinimide was obtained in the form of liver brown powder. Its yield to the theoretical value was 97%. Its molecular weight determined by GPC and calculated based on polystyrene was 15,000 in terms of weight average molecular weight and 8,800 in terms of number average molecular weight. Polysuccinimide thus obtained was hydrolyzed in the same manner as in Example 1 to obtain 3.6 g of yellowish white sodium polyaspartate. Its molecular weight determined by GPC and calculated based on polyethylene glycol was 16,000 in terms of weight average molecular weight and 8,900 in terms of number average molecular weight. Its calcium sequestration capacity was 4.2 ($Ca^{++}g/100$ g polymer).

COMPARATIVE EXAMPLE 4

The reaction was carried out in the same manner as in Example 1 using 25 g of aspartic acid as a starting material, 80 g of mesitylene as a solvent and no acid catalyst. Water did not form during the reaction and the degree of the conversion was almost 0%.

COMPARATIVE EXAMPLE 5

Into a 1-L eggplant flask were placed 50 g of aspartic acid and 30 g of 85% phosphoric acid. This eggplant flask was fit to a rotary evaporator and heated in an oil bath maintained at 180°C for 4 hours to carry out a polycondensation reaction. After the completion of the reaction, the eggplant flask was cooled and the glass-like lump thus formed was dissolved in N,N-dimethylformamide. Water was added thereto to form a precipitate which was collected by filtration.

The resulting precipitate was washed with water until it became neutral to remove phosphoric acid. The washed precipitate was dried at 85°C for 24 hours to obtain 17.1 g of yellowish white polysuccinimide powder. Its yield to the theoretical value was 47%. Its molecular weight thus obtained was 22,000 in terms of weight average molecular weight and 9,000 in terms of number average molecular weight.

INDUSTRIAL APPLICABILITY

The first polyaspartic acid or salts thereof according to the present invention has an excellent calcium sequestration capacity, and are useful as a chelating agent, a scale inhibitor, a builder for detergent, a dispersing agent, a humectant, an additive for fertilizer, various coating agents and the like. According to the process for producing the polyaspartic acid or salts thereof of the present invention, the polyaspartic acid and salts thereof can be simply produced in remarkably high yield as compared with the conventional methods.

According to the second process for producing the polyaspartic acid or salts thereof of the present invention, polysuccinimide having high molecular weight can be produced and, using it, high molecular weight polyaspartic acid having an excellent calcium sequestration capacity can be obtained. Further, the molecular weight can be controlled widely depending on its usage.

**Claims**

1. Polyaspartic acid or salts thereof having a calcium sequestration capacity determined by the calcium ion electrode method using calcium chloride of not less than 4.3 ($Ca^{++}g/100$ g polymer).

2. The polyaspartic acid or salts thereof as claimed in Claim 1, wherein said calcium sequestration capacity determined by the calcium ion electrode method using calcium chloride is not less than 4.6 ($Ca^{++}g/100$ g polymer).

3. A process for producing the polyaspartic acid or salts thereof according to Claim 1 which comprises a polycondensation step in which a monomer selected from the group consisting of a reaction product obtained by reacting maleic acid with ammonia, aspartic acid and maleamic acid is subjected to a polycondensation reaction in the presence of a solvent and an acid catalyst to obtain polysuccinimide, and a hydrolysis step in which polysuccinimide obtained in the polycondensation step is hydrolyzed.

4. The process as claimed in Claim 3, wherein said monomer is aspartic acid.

5. The process as claimed in Claim 3, wherein said solvent is selected from the group consisting of hydrocarbon type solvents, halogenated hydrocarbon type solvents, ether type solvents, ester type solvents and aprotic polar solvents.

6. The process as claimed in Claim 3, wherein said solvent has a boiling point of not less than 100°C.

7. The process as claimed in Claim 3, wherein said solvent has a boiling point of not less than 130°C.

8. The process as claimed in Claim 3, wherein said acid catalyst is used in an amount of from 0.0002 to 2 moles per mole of the monomer.

9. Polysuccinimide which is a precursor of the polyaspartic acid or salts thereof as claimed in Claim 1 and can be hydrolyzed to give said polyaspartic acid or salts thereof.

10. The polysuccinimide as claimed in Claim 9, which is obtained by subjecting a monomer selected from the group consisting of a reaction product obtained by reacting maleic acid with ammonia, aspartic acid and maleamic acid to a polycondensation reaction in the presence of a solvent and an acid catalyst.

11. A process for producing polyaspartic acid or salts thereof which comprises a polycondensation step in which a monomer selected from the group consisting of a reaction product obtained by reacting maleic acid with ammonia, aspartic acid and maleamic acid is subjected to a polycondensation reaction in the presence of an acid catalyst and a mixed solvent which comprises two or more solvents including at least one aprotic polar solvent to obtain polysuccinimide, and a hydrolysis step in which polysuccinimide obtained in the polycondensation step is hydrolyzed.

12. The process as claimed in Claim 11, wherein said monomer is aspartic acid.

13. The process as claimed in Claim 11, wherein each of said two or more solvents has a boiling point of not less than 100°C.

14. The process as claimed in Claim 11, wherein each of said two or more solvents has a boiling point of not less than 130°C.

15. The process as claimed in Claim 11, wherein said mixed solvent comprises an aprotic polar solvent and a solvent selected from the group consisting of hydrocarbon type solvents, halogenated hydrocarbon type solvents, ether type solvents and ester type solvents.

16. The process as claimed in Claim 11, wherein said acid catalyst is used in an amount of from 0.0002 to 2 moles per mole of the monomer.

17. The process as claimed in Claim 11, wherein, in said polycondensation step, the monomer is subjected to a polycondensation reaction in the presence of the solvent, the acid catalyst and primary amine or secondary amine.

18. The process for producing polyaspartic acid or salts thereof as claimed in Claim 17, wherein said primary amine or secondary amine has an acid dissociation constant (pKa) of not more than 7 in an aqueous solution.

## INTERNATIONAL SEARCH REPORT

| International application No. |
|---|
| PCT/JP95/02623 |

**A. CLASSIFICATION OF SUBJECT MATTER**

Int. Cl$^6$  C08G73/00, C08G73/10, C08G69/00, C08G69/10

According to International Patent Classification (IPC) or to both national classification and IPC

**B. FIELDS SEARCHED**

Minimum documentation searched (classification system followed by classification symbols)

Int. Cl$^6$  C08G69/00-C08G69/50, C08G73/00-C08G73/26

Documentation searched other than minimum documentation to the extent that such documents are included in the fields searched

| | |
|---|---|
| Jitsuyo Shinan Koho | 1926 - 1995 |
| Kokai Jitsuyo Shinan Koho | 1971 - 1995 |
| Toroku Jitsuyo Shinan Koho | 1974 - 1995 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP, 6-298930, A (Bayer AG.), | 1 - 10 |
| Y | October 25, 1994 (25. 10. 94), | 11 - 18 |
| | Claim, columns (0025), (0030) | |
| | & EP, 604813, A1 & DE, 4244031, A1 | |
| Y | JP, 49-011990, A (Ajinomoto Co., Inc.), | 11 - 18 |
| | February 1, 1974 (01. 02. 74), | |
| | Claim, line 19, right column, page 1 to line 13, | |
| | upper left column, page 2 (Family: none) | |
| Y | JP, 3-048628, A (Röhm G.m.b.H.), | 17, 18 |
| | March 1, 1991 (01. 03. 91), | |
| | Claim & US, 5175285, A & EP, 406623, A1 | |
| | & DE, 3921912, A1 | |
| A | JP, 6-211984, A (Rohm and Haas Co.), | 1 - 18 |
| | August 2, 1994 (02. 08. 94), | |
| | Claim & EP, 578451, A1 & US, 5371179, A | |
| A | JP, 6-248075, A (Bayer AG.), | 1 - 18 |
| | September 6, 1994 (06. 09. 94), | |

[X] Further documents are listed in the continuation of Box C.     [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| March 6, 1996 (06. 03. 96) | March 26, 1996 (26. 03. 96) |

| Name and mailing address of the ISA/ | Authorized officer |
|---|---|
| Japanese Patent Office | |
| Facsimile No. | Telephone No. |

Form PCT/ISA/210 (second sheet) (July 1992)

**INTERNATIONAL SEARCH REPORT**

| International application No. |
| --- |
| PCT/JP95/02623 |

C (Continuation). DOCUMENTS CONSIDERED TO BE RELEVANT

| Category* | Citation of document, with indication, where appropriate, of the relevant passages | Relevant to claim No. |
| --- | --- | --- |
| | Claim & EP, 612784, A1 & DE, 4319044, A1 | |
| A | JP, 59-112260, A (Andrew J. Arbart),<br>June 28, 1984 (28. 06. 84),<br>Claim & US, 4517241, A | 1 - 18 |

Form PCT/ISA/210 (continuation of second sheet) (July 1992)